Europäisches Patentamt

**European Patent Office**

Office européen des brevets

(11) Publication number: **0 010 865**
**B1**

# (12) EUROPEAN PATENT SPECIFICATION

(45) Date of publication of patent specification: **12.09.84**

(51) Int. Cl.³: **A 61 F 1/00, D 04 H 3/02**

(21) Application number: **79302041.3**

(22) Date of filing: **28.09.79**

(54) **Product adapted for transcutaneous use.**

(30) Priority: **10.10.78 GB 4002878**

(43) Date of publication of application:
**14.05.80 Bulletin 80/10**

(45) Publication of the grant of the patent:
**12.09.84 Bulletin 84/37**

(84) Designated Contracting States:
**CH DE FR GB IT NL SE**

(56) References cited:
**DE-A-2 423 338**
**DE-B-2 232 002**
**DE-B-2 312 457**
**FR-A-2 240 026**
**FR-A-2 340 079**
**GB-A-1 530 990**
**US-A-3 579 642**
**US-A-3 879 767**
**US-A-4 044 404**
**US-A-4 084 266**

(73) Proprietor: **IMPERIAL CHEMICAL INDUSTRIES PLC**
**Imperial Chemical House Millbank**
**London SW1P 3JF (GB)**

(73) Proprietor: **UNIVERSITY OF LIVERPOOL**
**Mount Pleasant PO BOX 147**
**Liverpool L69 3BX (GB)**

(72) Inventor: **Annis, David**
**"Little Hey" Dibbinsdale Road, Bromborough**
**Wirral, Merseyside (GB)**

(74) Representative: **Bate, Bernard James et al**
**Imperial Chemical Industries PLC Legal**
**Department: Patents PO Box 6**
**Welwyn Garden City Herts, AL7 1HD (GB)**

Courier Press, Leamington Spa, England.

## Description

This invention relates to products having a fibrillar surface area and to the production and use of such products.

A large variety of devices, structures, tubes, rods, fibres and other artifacts are used in medical and veterinary fields in locations where they come into intimate contact with cut surfaces of living tissues for example cut tissue surfaces, sometimes for extended periods of time, and it is desirable in such circumstances that the surfaces of such products, or at least a significant or appropriate portion of such surface of such a product is important in the living tissue and not isolated in the body for example by encapsulation. Acceptability of the surface of such a product is important is the case, for example, of a prosthetic device, where it may be desirable for surrounding tissues to grow into intimate contact with the prosthesis and to "bind" or hold it so that it effectively forms a part of the body. Such prostheses include incomponents which are employed to replace or supplement defective or deficient natural components of the body, but there are other devices or structures which may be located into the body for longer or shorter periods which it is desirable should be accepted by the living tissues. Examples of such include for example, irrigation tubes, leads to internal electrical or hydraulic structures, e.g. pacemakers, and the like. The skilled man will be familiar with the wide range of products which commonly are employed in association with the living body in the manner described above.

FR—A—2,240,026 and DE—B—2,312,457 for example relate to devices for transcutaneous location in the living body, the former describing specifically tubes for drainage of body liquids, the latter catheters, pacemaker leads and fibre optics. The said publications also refer to the provision, as an aid to contact of the device with the living tissue and the growth of tissue intimately with the device, of the presence of a fibrous cuff or tissue on the surface of the device.

We have now found that it is often desirable for the purpose of enhancing living tissue acceptability of products to be inserted into the body if at least a part of the surface of the product comprises a fibrous component having a small diameter, preferably a diameter that is relatively small in comparison with a living cell. Such fibres are conveniently not greater than 15 $\mu$m (i.e. 15 micron) in diameter. One method for the preparation of fibres having such small diameter is described for example in US—A—4,044,404, where the technique employed is that of electrostatic spinning.

Thus, the present invention provides a product as set out in claim 1.

Preferably the fibres are 0.5 to 10 $\mu$m in diameter and more preferably from 1 to 5 $\mu$m in diameter.

It will be appreciated that not all the said fibres need be of the dimensions stated above, but a significant proportion, conveniently greater than 10% preferably greater than 25% and more preferably greater than 50% or 70% and particularly preferably greater than 90% of such fibres will be of the stated dimensions.

The product itself may be fibrous in its entirety and it may comprise fibres of the same composition as those constituting its surface or part thereof as described above. All or some of such fibres comprising the bulk of the product may be of dimensions within the range previously specified or they may be of one or more different dimensions, for example where it is appropriate for the mode of application of the product or for reasons of mechanical or other property that the dimensions should be different. Alternatively the product may comprise a non-fibrous component which may be solid i.e. non-porous, porous or any other suitable quality. Thus, for example the product may be a composite product comprising at least two components, one of which is a fibrous component of characteristics hereinbefore set out. The second or further components may be of any appropriate material, texture and conformation to enable the product to perform its desired functions, and particular examples are set out hereunder. Thus, according to another aspect of the invention there is provided a product comprising a plurality of components, as set out in Claim 3.

With regard to the disposition of the fibres, while we do not necessarily exclude products in which the fibres of the fibrous surface component extend away from the mass of the product, that is, substantially at right angles to the mass of the product, as in the pile of velvet, it is preferred that the fibres of the fibrous component should lie so that their long axes are approximately parallel with the surface of the mass of the product. By approximately parallel it will be understood that we mean that the majority of such fibres are inclined at an angle of less than about 45° and preferably less than 30° to the most immediately adjacent surface of the product. The advantage of such a fibre disposition is that if and when cellular ingrowth between the fibres occurs the product will tend to be more firmly held by the ingrowing tissue than if the fibres extended at right angles to the adjacent surface.

We do not exclude the possibility of the fibres being woven though it is preferred that they are non-woven e.g. that they intermesh at random, although a wide range of disposition is acceptable; if desired both woven and non-woven components may be present.

The fibre component of the product may constitute part or all of the surface which will, when the product is located in the body, contact living cells of the body (this part of the surface of the product is referred to herein as the "contact surface"). The essential requirement is that the fibres should provide a product having greater acceptability to the body, improved adhesion properties to living tissue and encourages the skin to grow into the porous surface of an entire peripheral belt around the device to provide improved resistance to bacterial entry.

Formation of the fibrous component of the surface of the product may be by any convenient method. Thus the fibres may be preformed and applied to the surface of the product or the surface of the product may itself be formed into fibres by any convenient means. As examples of the former technique, we would mention formation of fibres, for example by drawing or spinning, including electrostatic spinning. Such fibres may then be applied to the surface of the product either as they are formed, or they may be collected to form for example a mat of appropriate thickness and the mat of fibres may then be applied to the surface of the product to provide the fibrous surface. An adhesive material may be applied to the surface of the product before application of the fibres, or part thereof, in order to facilitate adhesion, although particularly where the fibres are spun and applied to the surface while they retain some inherent adhesion the use of an adhesive may not be necessary. Again, the fibres may be drawn to the product for example by electrostatic forces, in which case adhesive may not be necessary. When an adhesive is employed it will, of course, be necessary to employ one which is appropriate for the intended use of the product.

The fibrous surface of the product preferably comprises a layer of fibres at least 5 $\mu$m and preferably at least 10 $\mu$m thick, conveniently 10 to 1000 $\mu$m preferably 25 to 500 $\mu$m, more preferably 30 to 250 $\mu$m and particularly 50 to 100 $\mu$m.

The distance between individual fibres will preferably be relatively small, for example it is preferred that the average inter-fibre distance in the fibrous surface does not exceed 100 $\mu$m and preferably most of the inter-fibre distances are of the order of 1 to 50 $\mu$m. Since fibres usually cross, this will provide an average pore diameter of the order of 1 to 50 $\mu$m preferably 1 to 15 $\mu$m and more preferably 2 to 5 $\mu$m.

Our preferred method of forming the fibres of the fibrous surface is by the so-called electrostatic spinning process.

The process of electrostatic spinning has been described in our French Application No. 77.03079 (FR—A—2340 079) and involves the introduction of a liquid into an electric field, whereby the liquid is caused to produce fibres which tend to be drawn to a charged collector. While being drawn from the liquid the fibres usually harden, which may involve mere cooling (where the liquid is normally solid at room temperature, for example), chemical hardening (for example by treatment with a hardening vapour) or evaporation of solvent (for example by dehydration). The product fibres may be collected on a suitably located and shaped collector which may be the product, the surface of which is to be rendered fibrous.

Fibres having different properties may be obtained by spinning different materials or by spinning a liquid containing a plurality of components, each of which may contribute a desired characteristic to the finished product, or by simultaneously spinning from different liquid sources fibres of different composition which are simultaneously deposited to form a matt having an intimately intermingled mass of fibres of different material. A further alternative is to produce a product having a plurality of layers of different fibres (or fibres of the same material but with different characteristics e.g. diameter) deposited, say, by varying with time the fibres deposited upon the receiving surface.

One way of effecting such a variation, for example, would be to have a moving receiver passing in succession sets of spinnerets from which fibres are being electrostatically spun, said fibres being deposited in succession as the receiver reaches an appropriate location relative to the spinnerets.

Materials suitable for the preparation of such fibres include polymeric substances and, in particular organic biologically inert, polymeric substances. By biologically inert we mean that the body is tolerant of the substance, which does not induce undesirable biological reaction. As preferred substances we would mention fluorinated hydrocarbons, e.g. polytetrafluoroethylene (PTFE) which conveniently may be spun from a dispersion of the material in a suitable dispersing agent, and polyurethanes which may be spun from solution.

The technique of electrostatic spinning provides a particularly convenient method of forming such surface layers to accord perfectly with the dimensions and contours of a desired product by making a major or non-fibrous component of the product a core upon which the fibrous material is collected.

The fibrous components of products of the present invention may be spun from a solution of or a dispersion of a polymer or its precursors. Polymers which may be conveniently spun from solution include high molecular weight fibreforming thermoplastics; in particular we would mention polyurethane, polyamides and polyacrylonitrile. Polymers which may conveniently be spun from dispersion include polytetrafluoroethylene and polyesters.

As an example of a polymer precursor which may be spun from solution we mention urea formaldehyde which may be cross-linked subsequent to spinning by treatment with acid vapour.

Water soluble polymes, e.g. polyvinyl alcohol, polyvinyl pyrrolidone and polyethylene oxide, may be spun from aqueous solution. While we do not exclude the possibility that products prepared from such materials may be used as prepared, preferably such products are given at least a degree of insolubility in aqueous medium e.g. by cross-linking with a suitable reagent.

Where the products are spun from a dispersion the spinning material comprises preferably also a solution of an additional component which acts to enhance the viscosity of the suspension and to improve its fibre forming properties. Most convenient for this purpose, we have found is an additional organic polymeric material which subsequent to fibre formation, can, if desired, be destroyed during sintering.

The preferred spinning material, then, is a solution of suspension which preferably comprises an organic polymer in an amount such that it is capable of forming a fibre and has cohesion properties such that the fibre form is retained during any post fibreization hardening until the fibre has hardened sufficiently not to lose its fibrous shape on detachment from a support where this is appropriate.

Where products are spun from dispersion they often have a tendency to be friable, being mere agglomerations of discrete particles held together in the form of fibres by the additional organic polymeric component present. Preferably such products are sintered so that the particles soften and flow into each other and the fibres may become point bonded. In the case of PTFE sintering may conveniently be carried out between 330°C and 450°C, preferably between 370°C and 390°C. Sterilisation may proceed concurrently during the sintering process. The sintering temperature in the case of PTFE is usually sufficiently high to destroy completely any undesirable organic component in the final product, e.g. material added solely to enhance viscosity or emulsifying agent.

The additional organic component need be employed only in a relatively small proportion (usually within the range 0.001 to 12% and preferably 0.1 to 3%) by weight of the suspension, although the precise concentration for any particular application can easily be determined by trial.

The degree of polymerisation of the additional organic component is preferably greater than about 2000 units linearly; a wide range of such polymers is available. An important requirement is solubility of the additional organic component in the selected solvent or suspending medium, which is preferably water. As examples of water-soluble additional organic compounds we may mention polyethylene oxide, polyacrylamide, polyvinyl pyrrolidone and polyvinyl alcohol; where an organic medium is employed to prepare the springing material, either as the sole liquid solvent or as a component thereof, a further wide range of organic polymeric compounds is available, for example polystyrene and polymethylmethacrylate.

The degree of polymerisation of the polymer will be selected in the light of required solubility and the ability of the polymer to impart the desired properties of cohesion and viscosity to the fibreizable liquid.

We have found that generally the viscosity of the fibreizable liquid whether due solely to the presence of the fibreizable polymer or partly contributed to by the additional organic polymer should be greater than $1 \times 10^{-2}$ Pa.s but not greater than 15 Pa.s. Preferably it is between $5 \times 10^{-2}$ to 5 Pa.s and more preferably between $1 \times 10^{-1}$ and 1 Pa.s (viscosities being measured at low shear rates). The viscosity required using a given additional organic polymer will vary with the molecular weight (MW) of the polymer, i.e. the lower the molecular weight the higher the final viscosity needed. Again, as the molecular weight of the polymer is increased a lower concentration of it is required to give good fibreization. Thus, as examples we would mention that in the preparation of polytetrafluoroethylene products we have found that using a polyethylene oxide of MW 100,000 as the additional organic polymer a concentration of about 12% by weight relative to the PTFE content is needed to give satisfactory fibreization, whereas with a MW of 300,000 a concentration of 1 to 6% may be adequate. Again, at a MW of 600,000 a concentration of 1 to 4% is satisfactory, while at a MW of $4 \times 10^6$ a concentration as low as 0.2% may give good fibreization.

The concentration of the fibreizable polymer will depend upon the amount required to provide adequate fibre properties, and will be influenced also by the need to produce a liquid of appropriate viscosity and speed of fibre hardening. Thus in the case of a dispersion we may use a concentration within the range 25% w/w to saturation (in the case of a dispersion, "saturation" means the maximum concentration which may be included without destroying the useful spinnability of the liquid) preferably 40 to 70% and more preferably 50 to 60%, and in the case of a solution we may use a concentration within the range 5% to 50% w/w, preferably 10 to 20% w/w.

It will be appreciated that the concentration of the components must each be adjusted to take account of the presence and concentration of any other and their relative effects upon viscosity, etc.

The spinning material should have some electrical conductivity, although this may vary between quite wide limits; for example we prefer to employ solutions having conductivity within the range $1 \times 10^{-4}$ to 5 S/m.

Any convenient method may be employed to bring the spinning material into the electrostatic field, for example we have supplied the spinning liquid to an appropriate position in the electrostatic field by feeding it to a nozzle from which it is drawn by the field, whereupon fibreization occurs. Any suitable apparatus can be employed for this purpose; thus we have fed the spinning material from a syringe reservoir to the tip of an earthed syringe needle, the tip being located at an appropriate distance from an electrostatically charged surface. Upon leaving the needle the material forms fibre between the needle tip and the charged surface.

Droplets of the spinning liquid may be introduced into the field in other ways which will be apparent to the skilled man, the only requirement being that they can be held within the field at a distance from the electrostatically charged surface such that fibreization occurs. For example they could be carried into the field on, say a continuous carrier, e.g. a metal wire.

It will be appreciated that where the liquid is fed into the field through a nozzle, several nozzles may be used to increase the rate of fibre production. Alternative means of bringing the fibreizable liquid into the charge field may be employed, for example a perforated plate (the perforations being fed with fibreizable liquid from a manifold) may be employed.

The optimum distance of the nozzle or other material supply means from the charged surface is determined quite simply by trial and error. We have found, for example, that using a potential of the order of 20 kV, a distance of 5—35 cm is suitable, but as the charge, nozzle dimensions, liquid flow rate, charged surface area etc. are varied so the optimum distance may vary, and it is most conveniently determined as described.

To allow high production rates, hardening of the fibres should occur rapidly and this is facilitated by the use of concentrated fibreizing liquids (so that the minimum liquid has to be removed), easily volatile solvents (for example the liquid may be wholly or partly of low boiling organic liquid) and relatively high temperatures in the vicinity of the fibre formation. The use of a gaseous, usually air, blast, particularly if the gas is warm, will often accelerate hardening of the fibre. Careful direction of the air blast may also be used to cause the fibres, after detachment, to lay in a desired position or direction.

The as-spun fibrous surface usually has a porosity in the range 5% to 95% and the porosity will depend on the particular application; a typical porosity value is 75%. By the term porosity we mean the percentage of the total volume of the fibrous component of the product which is free space.

Where dispersions are employed as the spinning material, the particle size may be between 0.1 $\mu$m and 1 $\mu$m preferably it is between .1 $\mu$m and .3 $\mu$m.

The application of products of the invention is in the provision of transcutaneous access from the exterior to the interior of the body. Such access is commonly required, sometimes for extended periods of time, and is commonly effected by means of catheters or other tubular devices, or by wires. Such access may be required for example in the case of renal failure to provide means for peritoneal dialysis; entry of electrical or other power conducting means, for example to energise internal devices e.g. artificial cardiac pacemakers, artificial muscles, artificial hearts, artificial kidneys, and any other such devices, or to energise or stimulate natural organs or structures e.g. nerve or muscle tissue in the body or to monitor operation of internal electrical devices or natural organs; access to the intestine for artificial feeding; access to blood vessels for injection of drugs, nutrients or removal of blood samples. Access to the body via the body wall is also often required for artificial removal of body contents, e.g. for drainage of urine or other body fluids, faeces, etc. Thus for example the invention could be employed in the provision of a colostomy device that would allow direct attachment of a collecting bag to a permanently implanted transcutaneous access device or the outlet of an internal artificial urinary bladder or artificial rectum.

It will be appreciated that in the case of many of the devices described above it may be necessary for the device to be provided with a fibrous surface over only a portion of its surface, i.e. at least where it contacts the cut skin, and accordingly such devices will commonly comprise a portion only part of the external surface of which is fibrous. Thus we have found that it may be sufficient to provide the device with a fibrous surface only in the region which will, when the device is in use be in the vicinity of the body surface. It is conjectured that the small diameter of the fibres of the fibrous material encourages intimate contact with and ingrowth to the fibrous surface of the tissues particularly of the body wall and that this contact, which may eventually become so intimate that the distance between tissue and fibre surface does not exceed $10^{-8}$ m, is sufficiently close to prevent entry of bacteria along the surface of the device into the body and hence tends to inhibit septic infection and ultimate rejection of the device from that cause. One consequence of the use of the product of the invention in such a location, therefore, may be to extend the period during which such a device may be located in the body without replacement. It is believed that the use of larger fibres than those we prefer would not allow such intimate contact with body tissues since the scar tissue cells are unable to ungulf the fibres and hence a gap is left between the fibre and the surrounding tissue large enough e.g. $10^{-6}$ m to allow penetration of bacteria. Thus, the fibrous surface of the product of the invention comprises fibres of diameter sufficiently small to enable them to be surrounded by scar tissue cells and ultimately for example connective tissue cells.

In such a location the advantage of devices of the invention is that when in contact with cells of a cut surface of the body ingrowth of tissue cells into the fibrous surface will be enhanced and in such a situation the cut surface will represent the living tissue of the body as used herein, i.e. the products of the invention comprise fibrous components upon a surface which when in use will contact the cut surface of living tissue.

Composite devices of the invention include for example, a fibrous surface component (preferably where the product is substantially tubular or hollow, such fibrous component being on an external surface) and a further component which may be a tube, rod, wire, which may be of e.g. polymer or metal, or a composite of different materials, and may be porous or permeable or non-porous or non-permeable.

The devices of the invention may comprise also ancillary structures or features appropriate to the use or application of the device. Examples include plugs or other closure means for tubular devices, attachment means, electrical connectors, amplifiers, dialysis equipment, needles etc.

For transcutaneous access it is often advantageous that the fibrous surface is of an area sufficient to contact substantially the cut surface of the body wall which has been cut to allow entry of the device. Preferably the fibrous surface is substantially confined to the surface area of the device which contacts such cut surface.

It will be apparent that where the product of the invention is a hollow composite the fibrous surface will usually be located on one side only of the material, for example in the case of a body access tube, the fibrous material will be located on the outer surface and will not contact liquid (e.g. blood) or other material flow through the tube.

The invention is further illustrated in the following examples.

### Example 1

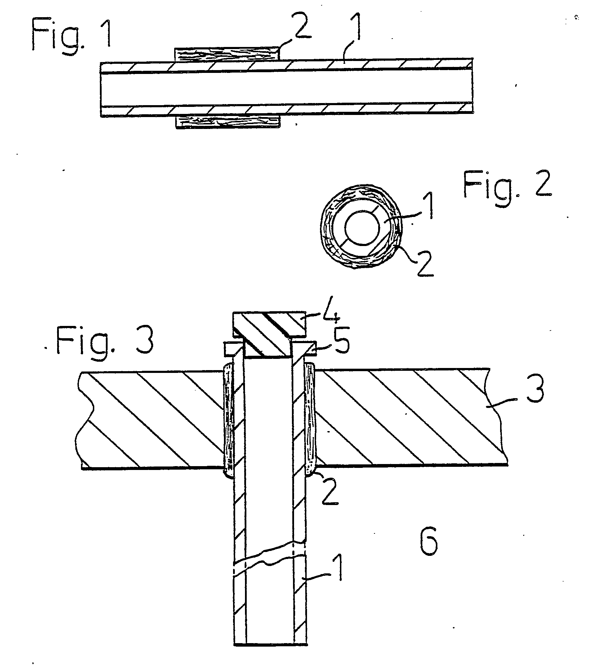

A transcutaneous device was prepared as followed. A mat of fibrous tissue 5 cm square and 1 mm thick was prepared as described by electrostatic spinning as described in US—A—4044404. The fibres of the mat were of polyurethane and of average diameter 5 μm. A portion of the mat was cut and was applied to the external surface of a 4 mm external diameter 40 cm long polyethylene tube by means of a non-toxic polyurethane adhesive to form a cuff about 18 mm long about 6 mm from one end of the tube. The tube (1) and attached cuff (2) are shown in longitudinal section in Figure 1 and in transverse section in Figure 2. In applying the cuff care was taken that the polyurethane adhesive solution was employed sufficiently sparingly so as to ensure that the adhesive did not so fill the inter-fibre spaces of the cuff as to prevent tissue ingrowth.

### Example 2

The tube prepared as described in Example 1 was employed as a transcutaneous device giving access to the peritoneal cavity to allow introduction of liquids into and removal of liquids from the peritoneal cavity (such as may be appropriate for renal peritoneal dialysis as in the case, for example, of renal failure).

The tube was inserted through an opening in the abdominal wall (the opening being slightly smaller than the diameter of the tube) the greater length of polyethylene tube being passed into the peritoneal cavity until the fibrous cuff lay adjacent the cut edges of the opening and was there held in place by means of a few prolene stitches. The shorter polyethylene end of the tube then projected out beyond the skin of the abdomen. A small nylon plug was placed in the end of the exposed tube to prevent direct access via the tube into the peritoneal cavity except as desired.

During the subsequent weeks tissue grew into the fibrous cuff, firmly securing and attaching it to to the track in the abdominal wall and because of the closeness with which the tissue came to surround each fibre of the cuff no infection occurred around the tube.

Subsequently the tube was removed with parts of the surrounding abdominal wall. Sections of the tube, cuff and associated surrounding scar tissue were prepared for light and electron microscopical examination. These sections showed a tissue ingrowth into the surface of the mesh such that the living connective tissue surrounded many of the fibres. There was not visible gap between the fibrous tissue and the polyurethane fibres of the cuff (the maximum resolution of the electron microscope was $10^{-9}$ m). It is concluded that the attachment of the connective tissue of the body wall to the fibrous cuff was so close that it prevented passage of bacteria between the tube and the surrounding living tissues, thereby preventing infection.

Figure 3 shows diagrammatically in section through the body wall and longitudinal to the device, a portion of the device in which 1 represents the polyethylene tube 2 the fibrous cuff, 3 the body wall, 4 the nylon plug. The tube is shown provided with an optional flange 5, 6 is the body cavity. The length of the cuff will depend upon the thickness of the body wall in the chosen location of penetration, as preferably the said length will be of the order of the thickness of the wall at that point since it may be disadvantageous to allow the cuff to extend to any substantial distance into the body cavity, and preferably it does not extend into the body cavity. A typical cuff length will be from 10 to 100 mm.

The diameter of the device will depend upon the particular intended application but it will range from say, 1 mm in the case, say, of a wire (where the fibrous component may conveniently be electrostatically spun directly onto the wire) through e.g. 10 mm for a dialysis tube, 20 mm for hydraulic lead tube, and 30 mm for a colostomy. Such dimensions are given merely as guides to the sizes of typical devices. The material and wall thickness of a tube will be selected in the light of the intended application and of the properties of the selected material. Thus it may be desired that it is rigid, in which case the wall may be relatively thick, or a rigid polymer may be employed or it may be desired that it be easily flexible or collapsible in which case thin and/or soft material may be used. Typical wall thicknesses of hollow devices of the invention will be from 0.5 mm to 3 mm preferably 1 to 2 mm.

## Claims

1. A product adapted for transcutaneous use in the body in which the outer surface of the product comprises a fibrous coating, characterised in that the coating is coextensive with at least the part of the outer surface of the product which is the intended site of union between the coating and living tissue in the vicinity of the body surface so that in use tissue growth into the coating will occur to hold the product and to provide a barrier to prevent bacteria entering the body along the surface of the product, and also in that the coating comprises fibres not greater than 15 microns in diameter.

2. A product according to claim 1 in which said fibres are between 0.5 and 10 $\mu$m in diameter.

3. A product according to claim 1 comprising a plurality of components, one of said components being a surface layer of fibres of diameter not greater than 15 $\mu$m.

4. A product according to claim 3 in which at least one component is a fibrous component having fibre greater than 15 $\mu$m in diameter.

5. A product according to claim 3 in which at least one component is non-fibrous.

6. A product according to claim 5 in which the non-fibrous component is a non-porous component.

7. A product according to claim 1 in which the surface fibres are disposed with their long axes approximately parallel with the surface of the mass of the product.

8. A product according to any of claims 1 to 7 in which the product is tubular.

9. A product according to claim 8 in which the product comprises an non-porous tubular component.

10. A product according to any of claims 1 to 7 in which the product is cylindrical.

## Patentansprüche

1. Erzeugnis, das für perkutane Verwendung im Körper geeignet ist, bei dem die Außenfläche des Erzeugnisses eine faserige Schicht enthält, dadurch gekennzeichnet, daß die Schicht die gleiche Ausdehnung hat wie mindestens derjenige Teil der Außenfläche des Erzeugnisses, der die gewünschte Stelle des Zusammenwachsens bzw. der Vereinigung der Schicht mit lebendem Gewebe in der Nähe der Körperoberfläche ist, so daß bei der Anwendung ein Gewebewachstum in die Schicht eintritt, um das Erzeugnis festzuhalten und eine Sperre zur Verhinderung des Eintretens von Bakterien in den Körper entlang der Oberfläche des Erzeugnisses zur Verfügung zu stellen, und daß die Schicht ferner Fasern mit einem Durchmesser von nicht mehr als 15 $\mu$m enthält.

2. Erzeugnis nach Anspruch 1, bei dem die Fasern einen Durchmesser zwischen 0,5 und 10 $\mu$m haben.

3. Erzeugnis nach Anspruch 1, das eine Vielzahl von Bestandteilen enthält, wobei einer der Bestandteile eine Oberflächenschicht aus Fasern mit einem Durchmesser von nicht mehr als 15 $\mu$m ist.

4. Erzeugnis nach Anspruch 3, bei dem mindestens ein Bestandteil ein faseriger Bestandteil ist, der Fasern mit einem Durchmesser von mehr als 15 $\mu$m enthält.

5. Erzeugnis nach Anspruch 3, bei dem mindestens ein Bestandteil nichtfaserig ist.

6. Erzeugnis nach Anspruch 5, bei dem der nichtfaserige Bestandteil ein nichtporöser Bestandteil ist.

7. Erzeugnis nach Anspruch 1, bei dem die Oberflächenfasern derart angeordnet sind, daß ihre Längsachsen zu der Oberfläche der Masse des Erzeugnisses annähernd parallel sind.

8. Erzeugnis nach einem der Ansprüche 1—7, wobei das Erzeugnis rohrförmig ist.

9. Erzeugnis nach Anspruch 8, wobei das Erzeugnis einen nichtporösen, rohrförmigen Bestandteil enthält.

10. Erzeugnis nach einem der Ansprüche 1—7, wobei das Erzeugnis zylinderförmig ist.

## Revendications

1. Produit conçu pour une utilisation transcutanée dans le corps, dans lequel la surface extérieure du produit comprend un revêtement fibreux, caractérisé en ce que le revêtement s'étend sur au moins une partie de la surface extérieure du produit qui constitue le site prévu de liaison entre le revêtement et le tissue vivant à proximité de la surface du corps afin que, lors de l'utilisation, une croissance du tissue vers l'intérieur du revêtement se produise pour maintenir le produit et pour établir une barrière empêchant la pénétration de bactéries dans le corps le long de la surface du produit, et également en ce que le revêtement comprend des fibres dont le diamètre n'est pas supérieur à 15 $\mu$m.

2. Produit selon la revendication 1 dans lequel lesdites fibres ont un diamètre compris entre 0,5 et 10 $\mu$m.

3. Produit selon la revendication 1 comprenant plusieurs éléments, l'un desdits éléments étant une couche superficielle de fibres d'un diamètre non supérieur à 15 $\mu$m.

4. Produit selon la revendication 3, dans lequel au moins un élément est un élément fibreux comportant des fibres de diamètre supérieur à 15 $\mu$m.

5. Produit selon la revendication 3, dans lequel au moins un élément est non fibreux.

6. Produit selon la revendication 5, dans lequel l'élément non fibreux est un élément non poreux.

7. Produit selon la revendication 1, dans lequel les fibres de surface sont disposées de façon que leurs grands axes soient approximativement parallèles à la surface de la masse du produit.

8. Produit selon l'une quelconque des revendications 1 à 7, dans lequel le produit est tubulaire.

9. Produit selon la revendication 8, dans lequel le produit comprend un élément tubulaire non poreux.

10. Produit selon l'une quelconque des revendications 1 à 7, dans lequal le produit est cylindrique.

0010865

Fig. 1

Fig. 2

Fig. 3

1